# EUROPEAN PATENT APPLICATION

(11) **EP 2 564 773 A1**
(43) Date of publication of application: **06.03.2013**
(21) Application number: 11179812.0
(22) Date of filing: 02.09.2011
(51) Int. Cl.: A61B 5/11, A61B 5/103, G06F 3/03

(54) **Method and apparatus for ergonomic evaluation of the postures of the wrist of an operator during a work cycle**

(71) Applicant: Fiat Group Automobiles S.p.A., 10135 Torino (TO) (IT); Politecnico di Torino, 10129 Torino (IT)
(72) Inventor: D'Aprile, Paola, 10043 Orbassano (Torino) (IT); Lamacchia, Sebastiano, 10043 Orbassano (Torino) (IT); Lionello, Davide, 10043 Orbassano (Torino) (IT); Marchetti, Paolo, 10129 Torino (IT)
(74) Representative: Notaro, Giancarlo

(57) **Abstract**

A method and an apparatus for ergonomic evaluation of the postures of the wrist (2) of an operator during a work cycle provide for the provision of sensor means (3, 4) for detecting the oscillation angle of the wrist both in the radio-ulnar oscillations plane and in the flexion-extension oscillations plane, and processing means (6) for monitoring, during the operation, the point for identifying the posture of the wrist (2) in a diagram bearing - on two cartesian axes - the values of the radio-ulnar oscillation angles and the values of the flexion-extension oscillation angles. The processing means (6) are adapted to send - to display means (7) - information regarding the percentage of the time for performing the work cycle in which the wrist (2) is located respectively in the following three parts of the diagram: the part of the diagram outside a first closed area (A), corresponding to unacceptable postures, the part of the diagram within a second closed area (B) which is in turn within said first closed area (A), said second closed area (B) corresponding to widely acceptable postures, and lastly the part of the diagram comprised between the perimeters of said first closed area (A) and said second closed area (B), corresponding to postures with intermediate level of acceptability.

## Description

The present invention refers to a method and an apparatus for ergonomic evaluation of the postures of the wrist of an operator during a work cycle.

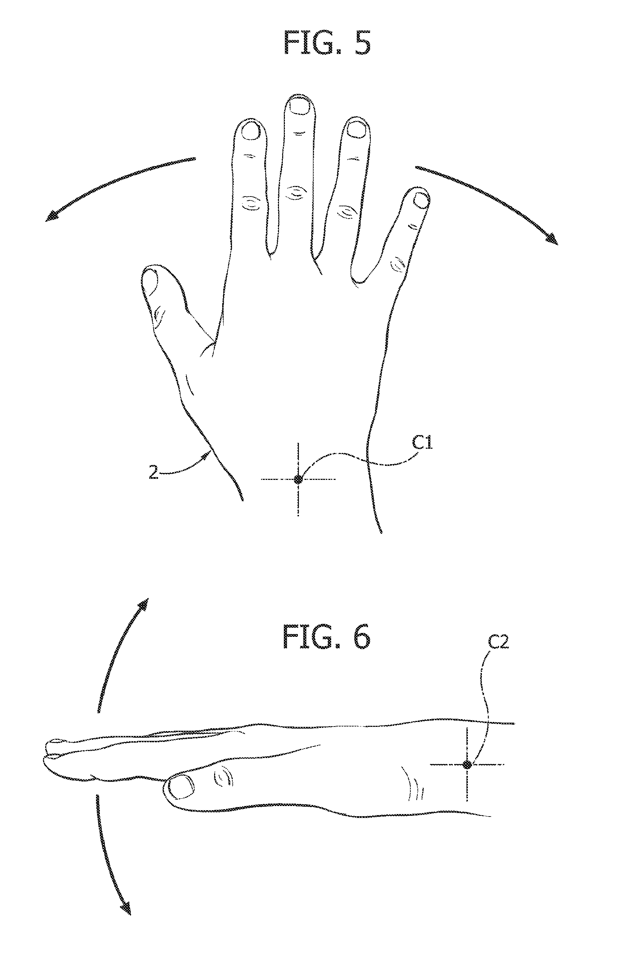

Over the last years there has been an ever-growing need for verifying whether the postures assumed by an operator during the work thereof are ergonomically acceptable and whether they are not harmful for the operator in question. Currently, there are regulations that provide for the direct observation of the postures assumed by the operator by trained and qualified people. However, there has been the need for providing instruments capable of performing such analysis in an accurate and reliable manner. Furthermore, with specific reference to monitoring the postures of the wrist, the methods implemented up to date consider the oscillations of the wrist of the radio-ulnar and flexion-extension type separately. The first type of oscillation is the one corresponding to a rotation of the hand in the plane of the palm (see figure 5 of the attached drawings). The flexion-extension oscillations are instead those in which the hand oscillates in a plane perpendicular to the plane of the palm (figure 6).

The object of the present invention is to provide a method and an apparatus capable of allowing performing instrument detections of the posture of the wrist of an operator during a work cycle, according to a method capable of guaranteeing accurate and reliable detection.

A further obj ect of the invention is to provide a method and an apparatus of the type indicated above also capable of providing an evaluation of the postures assumed by the wrist of the operator during the work cycle, from an ergonomic point of view, according to evaluation criteria that is more reliable with respect to the criteria used up to date.

With the aim of attaining such object, the invention aims at providing a method for ergonomic evaluation of the postures of the wrist of an operator during the performance of a work cycle, wherein:
- sensor means are provided for detecting the oscillation angle of the wrist both in the radio-ulnar oscillations plane and in the flexion-extension oscillations plane,
- processing means are provided for monitoring, during the operation, the point for identifying the posture of the wrist in a diagram bearing - on two cartesian axes - the values of the radio-ulnar oscillation angles and the values of the flexion-extension oscillation angles,

- said diagram being obtained having a first closed area, defining a first level of acceptability of the postures of the wrist, said first closed area being a rectangular area having the sides thereof corresponding to maximum admissible values of said radio-ulnar and flexion-extension oscillations, starting from a neutral position of the wrist,
- said diagram further being obtained having a second closed area within the first closed area and defining a second level of acceptability of the postures of the wrist,
- said second closed area being delimited by a substantially ellipsoid-shaped curve, obtained by small scaling, according to a predetermined ratio, a closed line defined as the locus of the points for identifying the maximum postures that can be achieved by the wrist in case of combined radio-ulnar and flexion-extension oscillations,
said processing means being adapted to send - to display means - information regarding the percentage of the time for performing the work cycle in which the wrist is respectively located in the following three parts of the diagram:
- the part of the diagram outside said first closed area, corresponding to unacceptable postures,
- the part of the diagram within said second closed area, corresponding to widely acceptable postures,
and the part of the diagram comprised between the perimeters of said first closed area and said second closed area, corresponding to postures with intermediate level of acceptability.

The invention also aims at providing an apparatus for implementing the method described above.

In the preferred embodiment, the aforementioned second closed area of the diagram for the reference and evaluation of the postures is obtained by scaling - by 50% - the closed line defined as the locus of the points for identifying the maximum postures that can be achieved by the wrist.

Further characteristics and advantages of the invention will be apparent from the description that follows with reference to the attached drawings, purely provided by way of non-limiting example, wherein:
figure 1 illustrates an operator during the performance of a work cycle,
figure 2 is a block diagram of the apparatus according to the invention,
figure 3 is a diagram for the reference and evaluation of the postures which is used in the method and in the apparatus according to the invention,
figure 4 illustrates an example of how the apparatus according to the invention displays the results of the performed analysis, and
figures 5, 6 are exemplifying figures showing the radio-ulnar and flexion-extension movements of the wrist of a hand.

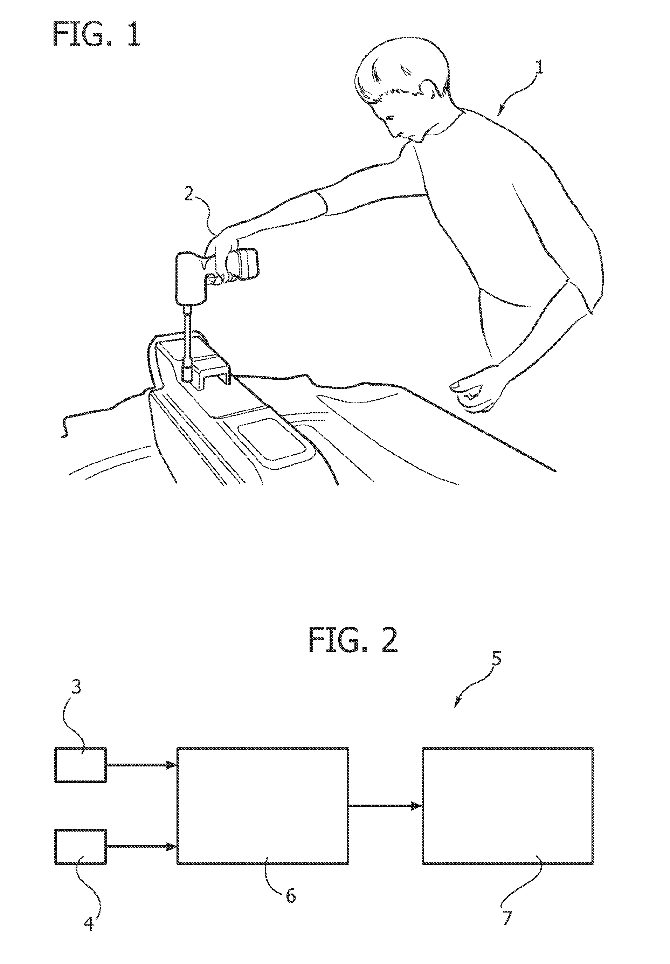

The method and apparatus according to the invention are provided for monitoring and evaluating - from an ergonomic point of view - the postures assumed by the wrist 2 of the hand with which an operator 1 (figure 1) performs a work cycle. The movements of the wrist that form an object of the analysis carried out through the method and the apparatus according to the invention are the radio-ulnar oscillations (see figure 5) and the flexion-extension oscillations (figure 6). The radio-ulnar oscillations are those in which the hand is rotated in the plane of the palm of the hand, around a centre of rotation C₁ defined by the wrist 2, starting from a neutral position (such oscillations are "radial" in one direction and "ulnar" in the other). The flexion-extension oscillations are those in which the hand is made to oscillate perpendicularly to plane of the palm (figure 6) through a rotation of the wrist in the vertical plane, around a centre C₂, starting from the neutral position (such oscillations are "flexion" oscillations in one direction and "extension" oscillations in the other).

According to the invention, there are provided one or more sensors 3,4 capable of monitoring the oscillation angle of the wrist 2 starting from the neutral position thereof, both in the radio-ulnar oscillations plane and in the flexion-extension oscillations plane. The sensors provided for such purpose may limitlessly vary in number and may be of any known type and they do not fall, considered on their own, within the scope of protection of the present invention. Hence, the construction details regarding the sensors are neither described nor illustrated herein. Such sensors can be constituted for example by an electro-goniometer of any type available in the market, or by optical sensors, or even for example by a glove worn by the operator and which carries sensors of any type (for example potentiometers) adapted to detect the relative position between the hand and wrist.

The apparatus according to the invention, indicated in its entirety with 5 in figure 2, comprises an electronic processing unit 6 which receives the signals of the sensors 3,4 during the cycle of operations performed by the operator and processes them to send - to display means 7 - the information regarding the postures assumed by the wrist 2 during the cycle of operations and the evaluation of such postures from an ergonomic point of view.

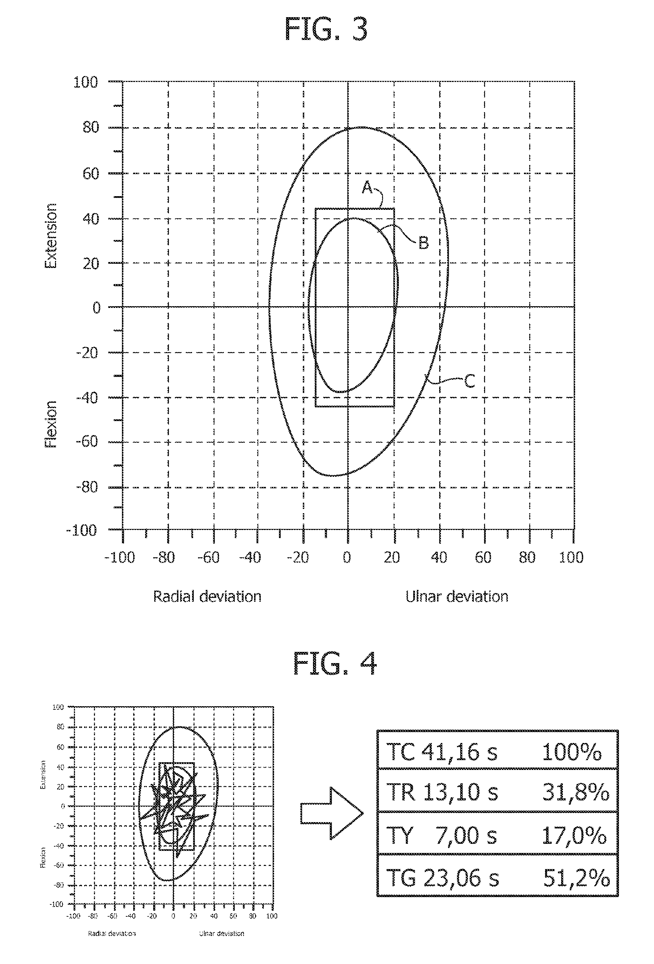

In order to provide such evaluation, the method according to the invention is based on a reference diagram illustrated in figure 3. In such diagram, there are indicated - in abscissa - the values of the oscillation angles in the radio-ulnar oscillations plane starting from a zero value corresponding to the neutral position of the wrist illustrated in figure 5. The positive values, on the right to zero on the axis of the abscissas of the diagram of figure 3, correspond to the ulnar oscillations, while the negative values correspond to radial oscillations. On the axis of the ordinates there are instead indicated the values of the oscillations in the flexion-extension plane (figure 6), the positive values corresponding to the extension oscillations and the negative values corresponding to the flexion oscillations. The method according to the invention provides for the display, in the diagram of figure 3, of a first closed area A, rectangular-shaped, defining a first level of acceptability of the postures of the wrist. The area A is delimited at the upper part and lower part by lines parallel to the axis of the abscissas, which correspond to the maximum admissible values of the flexion-extension oscillations. In the case of preferred embodiments such maximum admissible values are those provided for by the regulations in force (UNI EN 1005-5). The area A is limited on the right and left by two lines parallel to the axis of the ordinates corresponding to the maximum admissible values of the radio-ulnar oscillations. In the case of the preferred embodiment, such maximum admissible values are those defined by the regulations in force.

According to the invention, within the aforementioned first area A there is provided a second closed area B, substantially ellipsoid-shaped and defining a second level of acceptability of the postures of the wrist.

According to the invention, the profile of area B is obtained by small scaling (in case of the preferred embodiment it is scaled by 50%) a closed line C which is determined experimentally, as the locus of the points that define the maximum oscillations that can be achieved by the wrist. Each point of the perimeter line of the area C correspond to a position of the wrist determined by a combined oscillation of the radio-ulnar and flexion-extension type. As observable, should the radio-ulnar oscillation be close to zero, the flexion-extension oscillation may reach the maximum values thereof. On the contrary, should the two oscillations occur simultaneously, they penalise each other, hence for example, in the presence of a 30° ulnar deviation, the maximum extension oscillation that the wrist can achieve is lower than the absolute maximum which is close to 80°.

As indicated above, beginning from the experimental detection of the area C, according to the invention there is provided an area B, obtained by scaling - by 50% - area C and it is assumed that the postures of the wrist corresponding to points of the diagram within the area B are widely acceptable.

According to the invention, the display means 7 are capable of displaying the movement of the point for identifying the posture of the wrist in the diagram of figure 3, during a work cycle.

With reference to figure 4, the part to the left of such figure shows a representation of the diagram of the figure 3 as observable by display means 7 (for example in form of personal computer monitors), in which above areas A and B there remains the trace of the path covered by the point for identifying the posture of the wrist during the operation. The display means do not display the area C that - in the invention - is created solely for obtaining the area B and which has no function during the monitoring operation.

According to the areas A and B displayed on the diagram of the figure 3, the method and the apparatus according to the invention provide for a classification of the postures of the wrist according to the positioning of the point which identifies the posture on the diagram. Thus, the apparatus of the invention distinguishes the following three parts of the diagram:
- the part of the diagram outside the area A (named the "red" area), corresponding to definitely unacceptable postures;
- the part of the diagram within the area B (named the "green" area), corresponding to widely acceptable postures, and
- the part of the diagram comprised between the perimeters of the areas A and B (named the "yellow" area, corresponding to postures with intermediate level of acceptability.

The method and the apparatus according to the invention not only classify the postures assumed by the wrist during the operating cycle according to the aforementioned areas, but they also control the time at which the point for identifying the posture is located in the various areas of the diagram, indicated as percentage of the overall duration of the operating cycle. The display means 7 are thus capable of showing, at the end of the operation, a table of the type illustrated in the right part of figure 4, where the time is indicated in seconds corresponding to the overall duration of the operating cycle (TC), and the time at which the point for identifying the posture is in the "red" area (TR), in the "yellow" area (TY) and in the "green" area (TG), such time being indicated both as duration in seconds and as percentage of the time cycle.

Thus the apparatus and the method according to the invention are - on one hand - capable of accurately and reliably monitoring the postures of the wrist during the performance of the work cycle and - on the other hand - evaluating equally accurately and reliably, the quality of the operations assigned to the operator, from an ergonomic point of view.

Naturally, without prejudice to the principle of the invention, the construction details and the embodiments may widely vary with respect to what has been described and illustrated purely by way of example, without departing from the scope of protection of the present invention.

## Claims

1. Method for ergonomic evaluation of the postures of the wrist (2) of an operator (1) during the performance of a work cycle, wherein:
- sensor means (3, 4) are provided for detecting the oscillation angle of the wrist (2) both in the radio-ulnar oscillations plane and in the flexion-extension oscillations plane,
- processing means (6) are provided for monitoring, during the work cycle, the point for identifying the posture of the wrist (2) in a diagram bearing - on two cartesian axes - the values of the radio-ulnar oscillation angles and the values of the flexion-extension oscillation angles,
- said diagram being obtained having a first closed area (A), defining a first level of acceptability of the postures of the wrist (2), said first closed area (A) being a rectangular area having the sides thereof corresponding to maximum admissible values of said radio-ulnar oscillations and said flexion-extension oscillations, starting from a neutral position of the wrist (2),
- said diagram further being obtained having a second closed area (B) within the first closed area (A) and defining a second level of acceptability of the postures of the wrist (2),
- said second closed area (B) being delimited by a substantially ellipsoid-shaped curve, obtained by small scaling, according to a predetermined ratio, a closed lined (C) defined as the locus of the points for identifying the maximum postures that can be achieved by the wrist (2) in case of combined radio-ulnar and flexion-extension oscillations,
- said processing means (6) being adapted to send - to display means (7) - information regarding the percentage of the time for performing the work cycle in which the wrist (2) is located respectively in the following three parts of the diagram:
- the part of the diagram outside said first closed area (A), corresponding to definitely unacceptable postures,
- the part of the diagram within said second closed area (B), corresponding to widely acceptable postures, and
- the part of the diagram comprised between the perimeters of said first closed area (A) and said second closed area (B), corresponding to postures with intermediate level of acceptability.

2. Method according to claim 1, **characterised in that** said second closed area (B) is obtained by scaling - by 50% - the aforementioned closed line identifying the maximum postures that can be achieved by the wrist.

3. Apparatus for ergonomic evaluation of the postures of the wrist (2) of an operator during the performance of a work cycle, said apparatus comprising:
- sensor means (3, 4) for detecting the oscillation angle of the wrist (2) both in the radio-ulnar oscillations plane and in the flexion-extension oscillations plane,
- processing means (6) for monitoring, during the work cycle, the point for identifying the posture of the wrist (2) in a diagram bearing - on two cartesian axes
- the values of the radio-ulnar oscillation angles and the values of the flexion extension oscillation angles,
- said diagram being provided having a first closed area (A), defining a first level of acceptability of the postures of the wrist (2), said first closed area (A) being a rectangular area having the sides thereof corresponding to maximum acceptable values of said radio-ulnar and flexion-extension oscillations, starting from a neutral position of the wrist,
- said diagram further being provided having a second closed area (B) within the first closed area (A) and defining a second level of acceptability of the postures of the wrist (2),
- said second closed area (B) being delimited by a substantially ellipsoid-shaped curve, obtained by small scaling, according to a predetermined ratio, a closed line defined as the locus of the points for identifying the maximum postures that can be achieved by the wrist (2) in case of combined radio-ulnar and flexion-extension oscillations,
- said processing means (6) being adapted to send - to display means (7) - information regarding the percentage of the time for performing the work cycle in which the wrist (2) is located respectively in the following three parts of the diagram:
- the part of the diagram outside said first closed area (A), corresponding to definitely unacceptable postures,
- the part of the diagram within said second closed area (B), corresponding to widely acceptable postures, and
- the part of the diagram comprised between the perimeters of said first closed area (A) and said second closed area (B), corresponding to postures with intermediate level of acceptability.

4. Apparatus according to claim 3, **characterised in that** said second closed area (B) is obtained by scaling - by 50% - the aforementioned closed line (C) identifying the maximum postures that can be achieved by the wrist.
